# EUROPÄISCHE PATENTSCHRIFT

(11) **EP 2 870 191 B1**
(45) Veröffentlichungstag und Bekanntmachung des Hinweises auf die Patenterteilung: **15.08.2018**
(21) Anmeldenummer: 13727879.2
(22) Anmeldetag: 06.06.2013
(51) Int. Cl.: C08G 69/20

(54) **KATALYSATOREN FÜR DIE HERSTELLUNG VON GUSSPOLYAMID, VERFAHREN ZU DEREN HERSTELLUNG UND DEREN VERWENDUNG**
CATALYSTS FOR THE PRODUCTION OF CAST POLYAMIDE, METHOD FOR PRODUCING AND USING SAME
CATALYSEURS POUR LA FABRICATION DE POLYAMIDE DE FUSION, SON PROCÉDÉ DE FABRICATION ET SON UTILISATION

(30) Priorität: 06.07.2012 EP 12175416
(43) Veröffentlichungstag der Anmeldung: 13.05.2015
(73) Patentinhaber: LANXESS Deutschland GmbH, 50569 Köln (DE)
(72) Erfinder: LAUFER, Wilhelm, 67158 Ellerstadt (DE); UESTUENBAS, Serdar, 68169 Mannheim (DE)
(86) Internationale Anmeldenummer: PCT/EP2013/061726
(87) Internationale Veröffentlichungsnummer: WO 2014/005791

(56) Entgegenhaltungen:
- EP-A1- 2 447 302
- US-A- 3 663 670

## Beschreibung

Die vorliegenden Erfindung betrifft neue Katalysatoren für die Herstellung von Gusspolyamid, Verfahren zu deren Herstellung und deren Verwendung.

Gusspolyamide sind besonders hochmolekulare Polyamide. Bei der Herstellung von Gusspolyamiden wird ein Lactam zusammen mit mindestens einem Katalysator und mindestens einem Aktivator in eine Form gegossen und dann in dieser Form anionisch polymerisiert. Die in der Form vorliegenden Ausgangsverbindungen polymerisieren dabei im Allgemeinen unter Einwirkung von Wärme. Dabei entsteht ein homogener Werkstoff, welcher extrudierte Polyamide im Hinblick auf die Kristallinität übertrifft.

Gusspolyamide sind als thermoplastische Kunststoffe für die Fertigung komplexer Bauteile geeignet. Sie müssen im Gegensatz zu vielen anderen Thermoplasten nicht aufgeschmolzen werden, sondern entstehen durch anionische Polymerisation des Lactams in einer Form bei 120° bis 150°C bereits in wenigen Minuten. Dabei können alle bekannten Gießverfahren, wie Standguss, Spritzguss, Rotations- und Schleuderguss, angewendet werden. Als Endprodukt erhält man jeweils Formteile aus einem hochmolekularen, kristallinen Polyamid, das sich durch ein niedriges Gewicht, eine hohe mechanische Belastbarkeit, sehr gute Gleiteigenschaften und eine hervorragende Chemikalienbeständigkeit auszeichnet und das - da die Formen nicht unter Druck gefüllt werden - nur geringe innere Spannungen aufweist. Gusspolyamide lassen sich Sägen, Bohren, Fräsen, Schleifen, Verschweißen und Bedrucken oder Lackieren; neben komplexen Hohlformen werden aus diesem Polymer beispielsweise auch Rollen für Personenaufzüge und Halbzeuge, wie zum Beispiel Rohre, Stäbe und Platten für den Maschinenbau und die Automobilindustrie, gefertigt.

Die Herstellung von Gusspolyamidteilen, ausgehend von niedrig viskosen Lactamschmelzen und einem Katalysator sowie einem Aktivator, durch die sogenannte aktivierte anionische Polymerisation, ist an sich bekannt. Zu diesem Zweck werden üblicherweise zwei Mischungen aus Katalysator und Lactam bzw. Aktivator und Lactam in Form einer flüssigen Schmelze miteinander vermischt und anschließend in der Gussform polymerisiert, siehe EP-A-2447302. Aus US-A 3 663 670 ist ein Verfahren zur Polymerisation von Lactamen bekannt, wonach Lactam und Natriumhydrid in Gegenwart von Kaliumstearat umgesetzt und anschließend diese Mischung mit weiterem Lactam umgesetzt wird. Nachteilig bei den derzeitigen Katalysatoren für Gusspolyamide ist jedoch die zu hohe und nicht konstante Reaktivität. Dies führt zu schlechteren Produkteigenschaften, wie z. B. geringer Kristallinität, und macht das Procedere vor allem bei großen Gussteilen unwirtschaftlich.

Die Aufgabe der vorliegenden Erfindung bestand somit darin, neue Zusammensetzungen bereitzustellen, die sich als Katalysatoren bei der Herstellung von Gusspolyamiden eignen, und die die Nachteile des Standes der Technik nicht aufweisen.

Überraschenderweise wurde nun gefunden, dass die erfindungsgemäßen Zusammensetzungen dieses Eigenschaftsprofil aufweisen.

Gegenstand der vorliegenden Erfindung sind somit Zusammensetzungen enthaltend
a) mindestens ein Lactamat und
b) mindestens ein Salz einer mit Heteroatomen substituierten organischen Säure und/oder Ester einer mit Heteroatomen substituierten organischen Säure und gegebenenfalls
c) mindestens ein Lactam.

In einer bevorzugten Ausführungsform der Erfindung handelt es sich bei Lactamaten a) um mindestens eine Verbindung, ausgewählt aus der Gruppe der Alkali-Alumo-Dilactamate, Alkali- und/oder Erdalkalilactamate.

Bevorzugt als Lactamat im Sinne der Erfindung sind Alkali- und/oder Erdalkalilactamate, bevorzugt Natrium, Kalium und/oder Magnesium, einzeln oder im Gemisch.

Die vorgenannten Lactamate sind handelsüblich und sind beispielsweise bei der Firma Rhein Chemie Rheinau GmbH erhältlich.

In einer bevorzugten Ausführungsform der Erfindung handelt es sich bei dem Ester b) um C₁-C₆ - Alkylester, bevorzugt Methyl-, und/oder Ethylester. Die mit Heteroatomen substituierte organische Säure hat dabei vorzugsweise 1-12 Kohlenstoffatome, bevorzugt 4-6 Kohlenstoffatome.

In einer bevorzugten Ausführungsform der Erfindung hat das Salz einer mit Heteroatomen, vorzugsweise Aminogruppen, substituierten organischen Säure b) 1 - 12 -Kohlenstoffatome, besonders bevorzugt 4 - 6 Kohlenstoffatome.

In einer besonders bevorzugten Ausführungsform der Erfindung handelt es sich bei den Heteroatomen um Stickstoff, Schwefel, Phosphor, vorzugsweise Stickstoff, besonders bevorzugt Aminogruppen, und/oder um Halogenide, vorzugsweise Chlor und/oder Brom.

Besonders bevorzugt handelt es sich bei dem Salz einer mit Heteroatomen substituierten organischen Säure und/oder Ester einer mit Heteroatomen substituierten organischen Säure b) um Aminocapronate und/oder Aminolaurate, bevorzugt Alkali- und/ oder Erdalkaliaminocapronate und/oder -Laurate, besonders bevorzugt Natrium-, Kalium- und/oder Magnesium-Aminocapronat.

In einer bevorzugten Ausführungsform der Erfindung handelt es sich bei dem Lactam c) um eine Verbindung der allgemeinen Formel 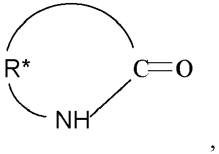 wobei R* eine Alkylengruppe mit 3 bis 13 Kohlenstoffatomen darstellt.

Bevorzugt sind dabei Capro-Lactam und/oder Laurin-Lactam. Diese sind kommerziell erhältlich, z.B. bei der Lanxess Deutschland GmbH.

Die erfindungsgemäße Zusammensetzung weist dabei vorzugsweise die Bestandteile a) und b) im Verhältnis 3:1 bis 50: 1, bevorzugt 5:1 bis 40: 1, besonders bevorzugt 9:1 auf.

Der Anteil an Lactam c), bezogen auf die Bestandteile a) und b) beträgt vorzugsweise 0 - 99 Gew.%, besonders bevorzugt 50 - 85 Gew.%.

Ein weiterer Gegenstand der Erfindung ist ein Verfahren zur Herstellung der erfindungsgemäßen Zusammensetzung bei dem das Lactamat a) durch Umsetzung von mindestens einem Lactam, welches Lactam c) entspricht, aber auch von diesem verschieden sein kann, mit Alkali- oder Erdalkali-Alkoholaten in Gegenwart von mindestens einem Salz einer mit Heteroatomen substituierten organischen Säure und/oder Ester einer mit Heteroatomen substituierten organischer Säure b) bei anschließender oder gleichzeitiger Entfernung des entstehenden Alkohols durch Destillation hergestellt und anschließend gegebenenfalls weiteres Lactam c) zugesetzt wird.

In einer bevorzugten Ausführungsform des erfindungsgemäßen Verfahrens wird das Lactamat a) durch Umsetzung von Lactam, bevorzugt Caprolactam, welches Lactam c) entspricht, aber auch von diesem verschieden sein kann, mit Na-Methanolat in Gegenwart von Na-Aminocapronat und/oder Na-Aminolaurat b) bei anschließender oder gleichzeitiger Entfernung des entstehenden Alkohols durch Destillation hergestellt und anschließend gegebenenfalls weiteres Lactam c) zugesetzt.

In einer weiteren bevorzugten Ausführungsform des erfindungsgemäßen Verfahrens wird das Lactamat a) durch Umsetzung von mindestens einem Caprolactam im Überschuss, welches Lactam c) entspricht, mit Na-Methanolat in Gegenwart von Na-Aminocapronat und/oder Na-Aminolaurat b) bei anschließender oder gleichzeitiger Entfernung des entstehenden Alkohols durch Destillation hergestellt.

Die vorgenannten Umsetzungen, d.h. die Herstellung des Lactamats sowie die Destillation, werden vorzugsweise bei Temperaturen von 80°- 130°C durchgeführt.

Das erfindungsgemäße Verfahren kann sowohl als Batch- als auch in einem Konti-Prozeß erfolgen. Dabei können die Salze einer mit Heteroatomen substituierten organischen Säure und/oder Ester einer mit Heteroatomen substituierten organischer Säuren vor, während und/oder auch nach der Herstellung des Lactamats a) zugegeben werden.

Ebenfalls bevorzugt ist die Verfahrensvariante, bei der die Salze einer mit Heteroatomen substituierten organischen Säure und/oder Ester der mit Heteroatomen substituierten organischen Säuren "in situ" entstehen. In einer bevorzugten Ausführungsform der Erfindung erfolgt die in situ Herstellung der Salze einer mit Heteroatomen substituierten organischen Säure und/oder Ester der mit Heteroatomen substituierten organischen Säuren b) durch Zugabe von Säuren, Wasser und/oder Alkali- und/oder Erdalkalihydroxiden zu a) und gegebenenfalls c). Besonders bevorzugt ist dabei die Zugabe von stöchiometrisch benötigten Wassermengen zur Einstellung der gewünschten Salz-Konzentrationen.

In einer weiteren Ausführungsform der Erfindung wird das Lactamat a) bei Temperaturen von 80 - 120°C gegebenenfalls zusammen mit Lactam c) aufgeschmolzen und mit mindestens einem Salz einer mit Heteroatomen substituierten organischen Säure und/oder Ester der mit Heteroatomen substituierten organischen Säure b), vorzugsweise unter Rühren, versetzt.

Für das Rühren können handelsüblich Rühraggregate, wie vorzugsweise Rührkessel oder Mischer, eingesetzt werden.

Ein weiterer Gegenstand der Erfindung sind Zusammensetzungen, erhältlich nach den vorgenannten erfindungsgemäßen Verfahren. In Bezug auf diesen Gegenstand der Erfindung wird auf die obigen Ausführungen, inklusive aller bevorzugten Ausführungsformen verwiesen.

Ebenso Gegenstand der Erfindung sind Gusspolyamide, erhältlich durch die Polymerisation von Lactamen mit mindestens einer erfindungsgemäßen Zusammensetzung aus a) und b) und gegebenenfalls c) bei Temperaturen von 80 - 180°C, vorzugsweise von 120 und 160°C, in Anwesenheit von Aktivatoren und gegebenenfalls weiteren Zusatz- und Hilfsstoffen.

Die Herstellung erfolgt vorzugsweise nach den dem Fachmann geläufigen Formgebungsverfahren, wie vorzugsweise Spritzguss-, Standguss- und/oder Rotationsguss.

Die Polymerisation von Lactamen erfolgt vorzugsweise durch Zugabe der erfindungsgemäßen Zusammensetzung in das bei Temperaturen von 80 - 120°C aufgeschmolzene Lactam und anschließender Zugabe einer Lactamschmelze mit mindestens einem Aktivator und weiteren Zusatz- und Hilfsstoffen.

Dabei erfolgt die Polymerisation zur Herstellung der Gusspolyamide vorzugsweise direkt in der Gießform.

Die Polymerisation erfolgt vorzugsweise unter Ausschluss von Luftfeuchtigkeit z. B. im Vakuum oder in inerter Atmosphäre.

Als Aktivatoren im Sinne der Erfindung können Isocyanate, Isocyanurate, Biurete, Allophanate, Uretdione und/oder Carbodiimide als Einzelverbindung oder in Form einer Mischung eingesetzt werden. Ebenfalls einsetzbar im Sinne der Erfindung sind Aktivatoren, die geblockt sind, z. B. mit Lactamen, besonders bevorzugt Caprolactam, mit Phenolen, Oximen und/oder Epoxiden ebenso wie lösemittelhaltige Aktivatoren. Als Lösemittel eignen sich: N-Alkylpyrrolidone, vorzugsweise, N-Methylpyrrolidon und N-Ethylpyrrolidon, Polyglycole,vorzugsweise Polyglycol DME 200, Dipropylenglycol DME oder Tetraethylenglykol DME.

Isocyanate im Sinne der Erfindung sind vorzugsweise Diisocyanate, besonders bevorzugt 2,4-Toluylendiisocyanat (TDI), 2,6-Toluylendiisocyanat, ein Gemisch aus 2,4-Toluylendiisocyanat und 2,6-Toluylendiisocyanat, Hexamethylen-1,6-diisocyanat, Cyclohexan-1,4-diisocyanat, Xylylendiisocyanat, Isophorondiisocyanat, 1,5-Naphthalindiisocyanat, 4,4'-Diphenylmethandiisocyanat, 2,4'-Diphenyl-methandiisocyanat, 2,2'-Diphenylmethandiisocyanat, 4,4'-Diphenyldimethyl-methandiisocyanat, 1,3-Phenylen-diisocyanat, 1,4-Phenylendiisocyanat, Dicyclohexylmethan-4,4'-diisocyanat, Dicyclohexylmethan-2,4'-diisocyanat, Dicyclohexylmethan-2,2'-diisocyanat, Methylcyclohexan-diisocyanat, Tetramethylxylylendiisocyanat, 2,6-Diisopropylphenylen-isocyanat und deren Gemische. Besonders bevorzugt ist Hexamethylen-1,6-diisocyanat. Die vorgenannten Verbindungen sind handelsüblich und sind beispielsweise bei der Firma Bayer MaterialScience AG erhältlich.

Isocyanurate im Sinne der Erfindung sind vorzugsweise Verbindungen der Formel (I) 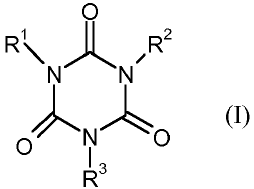
mit R¹, R² und R³ jeweils unabhängig voneinander -(CH₂)ₘ-N=C=O oder-(CH₂)_{q}-[(C₆H₃)(Me/Et)₃(N=C=O)] sind, und m = 1 - 12, q = 0 - 6 und Me für Methyl und Et für Ethyl steht, wobei
R¹, R² und R³ vorzugsweise gleich sind.

Dabei sind die folgenden Verbindungen der Formel (II) 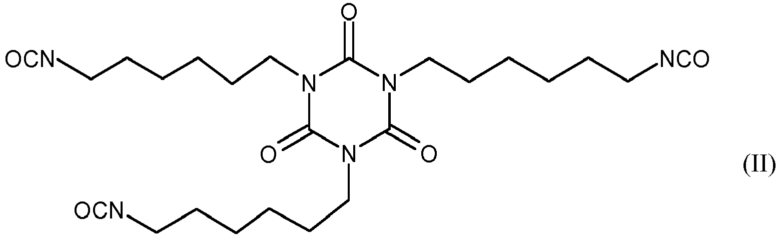 und der Formel (III) 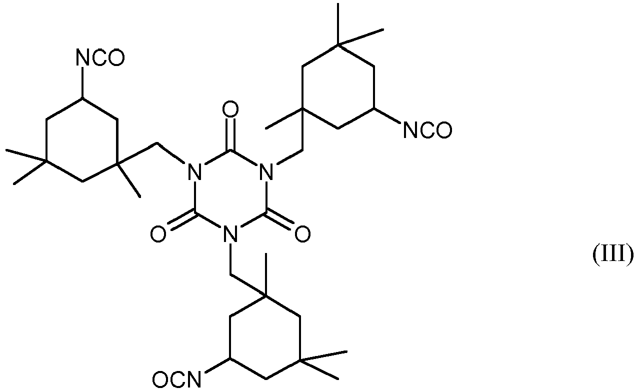 bevorzugt.

Die vorgenannten Verbindungen sind handelsüblich und sind beispielsweise bei der Firma Bayer MaterialScience AG erhältlich.

Biurete im Sinne der Erfindung sind vorzugsweise Verbindungen der Formel (IV) 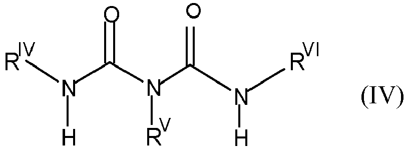
mit R^{IV}, R^{V} und R^{VI} jeweils unabhängig voneinander -(CH₂)ₚ₋N=C=O, mit p = 1 - 12, wobei
R^{IV} , R^{V} und R^{VI} vorzugsweise gleich sind.

In einer besonders bevorzugten Ausführungsform der Erfindung handelt es sich bei dem Biuret um eine Verbindung der Formel (V), d.h. einem Biuret der Formel (IV) mit R^{IV}, R^{V} und R^{VI} = -(CH₂)ₚ-N=C=O und p = 6

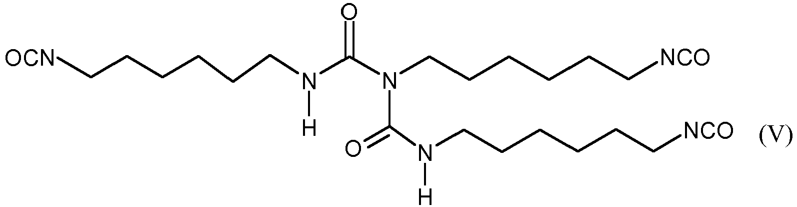

Die vorgenannten Verbindungen sind handelsüblich und sind beispielsweise bei der Firma Bayer MaterialScience AG erhältlich.

Uretdione im Sinne der Erfindung sind Umsetzungsprodukte von mindestens zwei Isocyanaten unter der Ausbildung von Dioxodiazetidin-Bindungen:

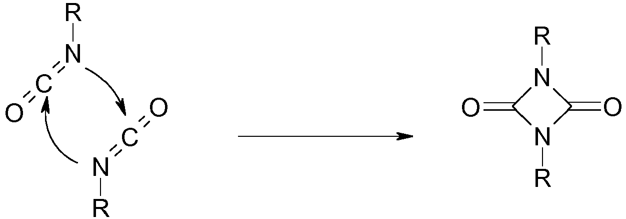

Die Herstellung ist dem Fachmann an sich bekannt. Die Verbindungen können beispielsweise gemäß der in EP 1 422 223 A1 beschriebenen Verfahren hergestellt werden.

Das Uretdion kann ein Dimer, Trimer, Oligomer oder Polymer sein.

Geeignete Beispiele für Uretdione sind dem Fachmann an sich bekannt. Bevorzugt sind Uretdione, die ausgehend von einem aliphatischen oder aromatischen Isocyanat erhalten werden. Die aromatischen Isocyanate weisen als R vorzugsweise 6 bis 20 Kohlenstoffatome, besonders bevorzugt 6 bis 15 Kohlenstoffatome, auf. Entsprechende aromatische monomere Isocyanate können beispielsweise ausgewählt werden aus der Gruppe, bestehend aus 2,4-Diisocyanatotoluol, 2,6-Diisocyanatotoluol, 1,5-Naphthylendiisocyanat, 4,4'-Methylen-diphenyldiisocyanat, 1,3-Bis-(3-isocyanato-4-methylphenyl)-2,4-dioxodiazetidin, N,N'-Bis-(4-methyl-3-isocyanatophenyl)-Harnstoff und Tetramethylxylylendiisocyanat. Von diesen aromatischen Isocyanaten sind 2,4-Diisocyanatotoluol, 2,6-Diisocyanatotoluol und 4,4'-Methylen-bis(phenyldiisocyanat) bevorzugt. Insbesondere bevorzugt sind 2,6-Diisocyanatotoluol und 4,4'-Methylen-bis(phenyldiisocyanat).

Die aliphatischen Isocyanate weisen als R vorzugsweise 6 bis 20 Kohlenstoffatome, besonders bevorzugt 6 bis 15 Kohlenstoffatome, auf. Entsprechende aliphatische monomere Isocyanate können beispielsweise ausgewählt werden aus der Gruppe, bestehend aus Isophorondiisocyanat, 1,4-Cyclohexyldiisocyanat, 1,1 -Methylen-bis-(4-isocyanatocyclohexan), 1,2-Bis-(4-isocyanatononyl)-3-heptyl-4-pentyl-cyclohexan und Hexamethylen-1,6-diisocyanat. Hierbei ist die Verwendung von Isophorondiisocyanat und Hexamethylen-1,6-diisocyanat bevorzugt.

Die vorgenannten Verbindungen sind handelsüblich und sind beispielsweise bei der Firma Rhein Chemie Rheinau GmbH oder die der Bayer MaterialScience AG erhältlich.

Allophanate im Sinne der Erfindung sind vorzugsweise Verbindungen der Formel (VI) 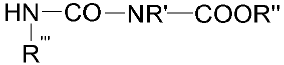 wobei R' und R" unabhängig voneinander ein Alkylrest mit 1 bis 20 Kohlenstoffatomen oder ein Arylrest mit 6 bis 20 Kohlenstoffatomen ist,
und R'" die Bedeutung eines Alkylrestes mit 1 bis 20 Kohlenstoffatomen hat

Diese Verbindungen sind im Allgemeinen durch Umsetzung beliebiger urethan- und/oder harnstoffgruppenhaltiger Ausgangsverbindungen, enthaltend Einheiten der allgemeinen Formel (R"OOC-NHR'), mit Monoisocyanaten der allgemeinen Formel R"'-NCO oder mit Diisocyanaten der allgemeinen Formel OCN-A-NCO zugänglich, wobei R'" bzw. A bevorzugt ein Alkylrest mit 1 bis 20 Kohlenstoffatomen oder ein Arylrest mit 6 bis 20 Kohlenstoffatomen ist und R' und R" unabhängig voneinander für Alkylreste mit 1 bis 20 Kohlenstoffatomen oder Arylreste mit 6 bis 20 Kohlenstoffatomen stehen.

Als Monoisocyanate eignen sich beliebige aromatische, aliphatische und cycloaliphatische Monoisocyanate mit bis zu 20 Kohlenstoffatomen, wie Methylisocyanat, Isopropylisocyanat, n-Butylisocyanat, n-Hexylisocyanat, Cyclohexylisocyanat, Stearylisocyanat, die gegebenenfalls halogenierten Phenylisocyanate, 1-Naphtylisocyanat, die gegebenenfalls chlorierten oder fluorierten m-, o-, und p-Toloylisocyanate, p-Isopropylphenylisocyanat, 2,6-Diisopropyl-phenylisocyanat und p-Toluolsulfonyldiisocyanat.

Als Diisocyanate eignen sich beliebige aromatische, aliphatische und cycloaliphatische Diisocyanate mit 6 bis 40 Kohlenstoffatomen, vorzugsweise 6 bis 15 Kohlenstoffatomen, wie besonders bevorzugt Isophorondiisocyanat, 1,4-Cyclohexyldiisocyanat, 1,1-Methylen-bis-(isocyanatohexan), 1,2-Bis-(4-Isocyanatononyl)-3-heptyl-4-pentylcyclohexan, Hexamethylen-1,6-diisocyanat, 2,4-Diisocyanatotoluol, 2,6-Diisocyanatotoluol, 1,5-Naphthylendiisocyanat, 4,4'-Methylen-diphenyldiisocyanat, 1,3-Bis-(3-isocyanto-4-methylphenyl)-2,4-dioxodiazetidin, N,N'-Bis-(4-methyl-3-isocyanatophenyl)-Harnstoff und Tetramethylxylylendiisocyanat. Bevorzugt hiervon ist Hexamethylen-1,6-diisocyanat.

Die im Rahmen der vorliegenden Erfindung besonders bevorzugten Allophanate sind Verbindungen der Formel (VII) 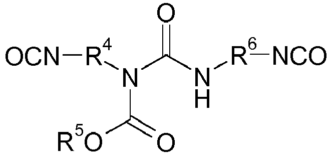 wobei R⁴ und R⁶ innerhalb des Moleküls gleich oder verschieden sein können und C₁-C₆-Alkyl, vorzugsweise -(CH₂)₆- bedeuten, und R⁵ C₁-C₆-Alkyl entspricht.

Entsprechende Allophanate sowie deren Herstellung sind beispielsweise in der EP 0 000 194A beschrieben, dessen diesbezügliche Offenbarung in die vorliegende Erfindung unter Bezugnahme eingeschlossen wird. Die vorgenannten Verbindungen sind handelsüblich und sind beispielsweise bei der Firma Bayer MaterialScience AG erhältlich.

Carbodiimide im Sinne der Erfindung sind vorzugsweise Verbindungen der Formel (VIII)

R¹¹-(-N=C=N-R¹²⁻)ₘ-R¹³ (VIII),

in der
m einer ganzen Zahl von 1 bis 500 entspricht,
R¹² für C₁-C₁₈-Alkylen, C₅-C₁₈-Cycloalkylen-, Arylen und/oder C₇-C₁₈- Aralkylen steht
R¹¹ für R¹²-NCO, R¹²-NHCONHR⁹, R¹²-NHCONR⁹R⁷ oder R¹²-NHCOOR⁸ steht und
R¹³ steht -NCO, -NHCONHR⁹, -NHCONR⁹R⁷ oder -NHCOOR⁸ steht,
wobei in R¹¹ unabhängig voneinander R⁹ und R⁷ gleich oder verschieden sind und einen C₁-C₆-Alkyl-, C₆-C₁₀-Cycloalkyl- oder C₇-C₁₈-Aralkylrest darstellen und R⁸ eine der Bedeutungen von R¹¹ hat oder einen Polyester- oder Polyamidrest oder -(CH₂)ₕ-O-[(CH₂)ₖ-O]_{g}-R¹⁰ entspricht,
mit h = 1-3, k= 1-3, g = 0-12, wobei
R¹⁰ die Bedeutung von H oder C₁-C₄-Alkyl hat.

Ebenfalls einsetzbar sind auch Gemische von Carbodiimiden der Formel (VIII), inklusive der entsprechenden Oligomere und/oder Polymere, wobei polymere Carbodiimide bevorzugt sind.

Die Verbindungen nach Formel (VIII) sind kommerziell erhältlich, z.B. bei der Firma Rhein Chemie Rheinau GmbH oder lassen sich nach den dem Fachmann geläufigen Verfahren herstellen, wie z.B. beschrieben in DE-A-11 30 594 oder US-A 2 840 589 oder durch die Kondensation von Diisocyanaten unter Abspaltung von Kohlendioxid bei erhöhten Temperaturen, z.B. bei 40 °C bis 200 °C, in Gegenwart von Katalysatoren. Als Katalysatoren haben sich vorzugsweise starke Basen oder Phosphorverbindungen bewährt. Bevorzugt werden Phospholenoxide, Phospholidine oder Phospholinoxide sowie die entsprechenden Sulfide verwendet. Ferner können als Katalysatoren tertiäre Amine, basisch reagierende Metallverbindungen, Carbonsäuremetallsalze und nicht basische Organometallverbindungen verwendet werden.

Die vorgenannten Verbindungen sind handelsüblich und sind beispielsweise bei der Firma Rhein Chemie Rheinau GmbH erhältlich.

Geblockte Aktivatoren, vorzugsweise mit Lactamen, besonders bevorzugt Caprolactam, oder mit Phenolen, Oximen und/oder Epoxiden geblockte Aktivatoren sind beispielsweise herstellbar über die Umsetzung mindestens einer Verbindung der Formeln (I) bis (VIII) mit mindestens einem Lactam, Caprolactam, Phenol, Oxim und/oder Epoxid bei Temperaturen von 80 bis 100°C nach den, dem Fachmann geläufigen Verfahren.

Zur Herstellung der Gusspolyamide werden in einer Ausführungsform der Erfindung, bezogen auf Lactam, vorzugsweise folgende Anteile eingesetzt:
0,1 bis 3 Gew.%, bevorzugt 0,2 bis 1,5 Gew.% der erfindungsgemäßen Zusammensetzung und 0,1 bis 2 Gew.%, bevorzugt 0,5 bis 1 Gew.% an Aktivator.

In einer bevorzugten Ausführungsform der Erfindung ist das Gusspolyamid erhältlich durch die Polymerisation von Lactam, vorzugsweise Caprolactam, mit
0,1 bis 3 Gew.% der erfindungsgemäßen Zusammensetzung enthaltend Natriumlactamat a) als vorzugsweise 18-20 Gew.%ige Caprolactam-Lösung mit 0,1 bis 5 Gew.%, bevorzugt, 0,5 - 4 Gew.%, besonders bevorzugt 1,5 - 2,5 Gew.% Na-Aminocapronat und
0,1 bis 2 Gew.% mindestens eines Vertreters, ausgewählt aus der Gruppe Hexamethylen-1,6-diisocyanat, mit Caprolactam geblocktem Hexamethylen-1,6-diisocyanat, Biuret der Formel (V) mit p = 6 und/oder einem Uretdion aus Basis von 2,4-Diisocyanatotoluol als Aktivator.

In einer weiteren bevorzugten Ausführungsform der Erfindung enthält die Mischung zur Herstellung von Gusspolyamiden zusätzlich mindestens einen weiteren Zusatz- und Hilfsstoff, ausgewählt aus Füll- und/oder Verstärkungsstoffen, Polymeren und/oder weiteren Zusatzstoffen.

Füll- und/oder Verstärkungsstoff im Sinne der Erfindung sind organische oder anorganische Füll- und/oder Verstärkungsstoffe. Bevorzugt sind anorganische Füllstoffe, insbesondere Kaolin, Kreide, Wollastonit, Talkum, Calciumcarbonat, Silikate, Titandioxid, Zinkoxid, Graphit, Graphene, Glaspartikel (z.B. Glaskugeln), nanoskalige Füllstoffe, (wie Kohlenstoff-Nanoröhren carbonanotubes), carbon black, Schichtsilikate, nanoskalige Schichtsilikate, nanoskaliges Aluminiumoxid (Al₂O₃), nanoskaliges Titandioxid (TiO₂) und/oder nanoskaliges Siliciumdioxid (SiO₂).

Weiterhin bevorzugt ist der Einsatz von Faserstoffen als Füll- und/oder Verstärkungsstoff. Die Füll- und/oder Verstärkungsstoffe werden in der Regel ausgewählt aus der Gruppe umfassend Mineralien in für Thermoplastanwendungen üblicher Korngröße, insbesondere Kaolin, Kreide, Wollastonit oder Talkum, Kohlenstoff- oder Glasfasern, vorzugsweise gemahlene Glasfasern, besonders bevorzugt Glas- und Kohlenstofffasern.

Besonders bevorzugt kommen ein oder mehrere Faserstoffe zum Einsatz, ausgewählt aus bekannten anorganischen Verstärkungsfasern, insbesondere Borfasern, Glasfasern, Kohlenstofffasern, Kieselsäurefasern, Keramikfasern und Basaltfasern; organischen Verstärkungsfasern, insbesondere Aramidfasern, Polyesterfasern, Nylonfasern, Polyethylenfasern; und Naturfasern, insbesondere Holzfasern, Flachsfasern, Hanffasern und Sisalfasern. Insbesondere bevorzugt ist der Einsatz von Glasfasern, insbesondere Schnittglasfasern, Kohlenstofffasern, Aramidfasern, Borfasern, Metallfasern und/oder Kaliumtitanatfasem.

Insbesondere können auch Mischungen der genannten Füll- und/oder Verstärkungsstoffe eingesetzt werden. Besonders bevorzugt werden als Füll- und/oder Verstärkungsstoffe Glasfasern und/oder Glaspartikel, insbesondere Glaskugeln, ausgewählt.

Die Menge an einzusetzenden Füll- und/oder Verstärkungsstoffen beträgt vorzugsweise 30 bis 90 Gew.-%, insbesondere von 30-80 Gew.-%, bevorzugt 30 bis 50 Gew.-%, weiterhin bevorzugt von 50 bis 90 Gew.-%.

Polymere im Sinne der Erfindung sind: Polystyrol, Styrol-Copolymersiate, insbesondere Styrol-Acrylnitril-Copolymere (SAN), Acrylnitril-Butadien-Styrol-Copolymere (ABS) oder StyrolButadien-Copolymere (SB), Polyphenylenoxidether, Polyolefine, insbesondere Polyethylen (HTPE (high-temperature-polyethylene), LTPE (low-temperature-polyethylene), Polypropylen oder Polybuten-1, Polytetrafluorethylen, Polyester, insbesondere Polyethylenterephthalat (PET); Polyamide, Polyether, insbesondere Polyethylenglykol (PEG), Polypropylenglykol oder Polyethersulfone (PESU oder PES); Polymere aus Vinylgruppen enthalten Monomeren, insbesondere Polyvinylchlorid, Polyvinylidenchloride, Polystryrol, schlagzäh-modifiziertes Polystyrol, Polyvinylcarbazol, Polyvinylacetat oder Polyvinylalkohol, Polyisobutylene Polybutadien und/oder Polysulfone. Es ist weiterhin die Verwendung von Copolymeren als Polymer möglich, die aus den Monomereinheiten der oben genannten Polymere bestehen.

In einer weiteren Ausführungsform der Erfindung kann das einzusetzende Polymer Gruppen enthalten, die zur Bildung von Block- und/oder Pfropf-Copolymeren mit dem aus den Monomeren gebildeten Polymeren, geeignet sind. Beispiele für solche Gruppen sind Epoxy-, Amin-, Carboxyl-Anhydrid-, Oxazolin-, Carbodiimid-, Urethan-, Isocyanat- und Lactam-Gruppen. Polymere mit Carbodiimid-Gruppen werden dann eingesetzt, wenn kein Carbodiimid als Aktivator eingesetzt wird.

Optional enthaltene Polymer ist vorzugsweise in einer Menge von 0 bis 40 Gew.-%, bevorzugt von 0 bis 20 Gew.-%, besonders bevorzugt in einer Menge von 0 bis 10 Gew.-% enthalten.

In einer bevorzugten Ausführungsform enthält erfindungsgemäße Zusammensetzung weitere Zusatzstoffe. Bevorzugt werden die Zusatzstoffe in einer Menge von 0 bis 5 Gew.-%, besonders bevorzugt von 0 bis 4 Gew.-%, ganz besonders bevorzugt von 0 bis 3,5 Gew.-% eingesetzt. Als Zusatzstoffe können bevorzugt Stabilisatoren, insbesondere Kupfersalze, Farbstoffe, Antistatika, Füllöle, Stabilisatoren, Oberflächenverbesserer, Sikkative, Entformungs-Hilfsmittel, Trennmittel, Antioxidantien, Lichtstabilisatoren, PVC-Stabilisatoren, Gleitmittel, Polyole, Flammschutzmittel, Treibmittel, Schlagzähmodifikatoren und/oder Nukleierungshilfsmittel zugesetzt werden.

Als Schlagzähmodifikatoren sind insbesondere Polydien-Polymere, vorzugsweise Polybutadien, Polyisopren, enthaltend Anhydrid und/oder Epoxigruppen geeignet. Das Polydien-Polymer weist eine Glasübergangstemperatur unter 0 °C, bevorzugt unter -10 °C, besonders bevorzugt unter -20°C auf. Das Polydien-Polymer kann auf der Basis eines Polydien-Copolymers mit Polyacrylaten, Polyethylenacrylaten und/oder Polysiloxanen basieren und mittels der gängigen Verfahren hergestellt werden, vorzugsweise durch Emulsionspolymerisation, Suspensions-polymerisation, Lösungspolymerisation, Gasphasenpolymerisation.

In einer weitern bevorzugten Ausführungsform der Erfindung wird als Zusatzstoff Polyol eingesetzt, um die Schlagzähigkeit zu verbessern. Diese sind z.B. erhältlich von der Rhein Chemie Rheinau GmbH unter der Bezeichnung Addonyl® 8073. Ebenfalls einsetzbar sind Polyoltriamine geeignet, um die Tieftemperatur-Schlagzähigkeit zu verbessern. Ein geeignetes Produkt ist Addonyl® 8112. Vorzugsweise werden die Polyole im Konzentrationsbereich 1-20 Gew.-% eingesetzt.

Die optionale Zugabe von Füll- und/oder Verstärkungsstoffen und weiteren Zusatzstoffen kann vor oder zusammen mit der Zugabe von Katalysator und/oder Aktivator erfolgen.

In einer weiteren Ausführung der vorliegenden Erfindung kann die Polymerisation zur Herstellung von Gusspolyamiden nach einem geeigneten Formgebungsverfahren, vorzugsweise Spritzguss, Standgussverfahren, Rotationsgussverfahren, durchgeführt werden.

Bei Spritzguss, Standgussverfahren, Rotationsgussverfahren handelt es sich um dem Fachmann geläufige Verfahren.

Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Ein weiterer Gegenstand der vorliegenden Erfindung ist die Verwendung der erfindungsgemäßen Zusammensetzung als Katalysator zur Herstellung von Gusspolyamid.

Darüber hinaus ist die Verwendung der erfindungsgemäßen der Zusammensetzung zur Herstellung von Rollen, vorzugsweise für Personenaufzüge und Halbzeuge, vorzugsweise Behälter, Zahnräder, Rohre, Stäbe und Platten für den Maschinenbau und die Automobilindustrie ein weiteren Gegenstand der Erfindung.

Die nachfolgenden Beispiele dienen der Erläuterung der Erfindung, ohne dabei limitierend zu wirken.

### Ausführungsbeispiele:

### Reagenzien:

Caprolactam trocken (EP > 69 °C) von der Firma Lanxess Deutschland GmbH
Aktivator, ein Hexamethylen-1,6-Diisocyanat (HDI)-Biuret, 70% in N-Ethylpyrrolidon, kommerziell erhältlich bei der Rhein Chemie Rheinau GmbH,
Erfindungsgemäße Zusammensetzung als Katalysator (A) mit ca. 18 % Natriumcaprolactamat und 2,0 % Natriumaminocapronat in Caprolactam.

Zusammensetzung nach dem Stand der Technik als Katalysator (B) mit ca. 18 % Natriumcaprolactamat in Caprolactam, Vergleich.

### Geräte:

Die verwendete Apparatur zur Schmelzaufbereitung bestand aus:
- 2 Dreihalskolben (500 ml) im Ölbad beheizt
- 2 KPG-Rührer mit Hülse
- 2 Gaskappen je 1 mit und 1 ohne Hahn
- 1 Vakuumpumpe mit Kühlfalle und Manometer.

Die verwendete Apparatur zur Temperaturmessung bestand aus:
- Temperaturmessgerät Testo 175-T3 mit IR-Serial Interface
- Thermodraht zum Verbleib in der ausgehärteten Probe
- Becherglas 600 ml (hohe Form) und einer
- Beheizung für das Becherglas (Metallblock, Ölbad).

### Durchführung und Messung der Topfzeit:

Kolben A wurde mit 196,8 g Caprolactam und 3,2 g Aktivator, Kolben B1/2 mit 192 g Caprolactam und 8 g Katalysator (A) bzw. (B) beschickt.

Die Schmelzen aus den Kolben A und Kolben B1/2 wurden bei 110 - 130 °C (± 2 °C) in einem Ölbad unter Vakuum (<15 mbar) 20 Minuten aufbereitet.

Nach Belüften mit Stickstoff wurden Komponenten aus Kolben A und Kolben B in einen Dreihalskolben vereinigt, kurz durchgerührt und in das 600 ml Becherglas überführt.

Die Formtemperatur (Becherglas) betrug 160° C. Die Polymerisationszeit betrug in der Regel 10 - 20 Minuten.

Die Trübungstest erfolgten im Trockenschrank 85 °C nach einem Zeitraum von 3 h. Die Beurteilung erfolgte visuell.

Die Beurteilung der Qualität der Gusspolyamide in Bezug auf Kristallinität und Homogenität erfolgte anhand von REM-Aufnahmen (Raster-Elektronen-Mikroskop).

| Katalysator in Kolben B1/2 | Topfzeit | Trübungstest | Kristallinität/Homogenität |
|---|---|---|---|
| | (s) | (85 °C, 3 h) | Gusspolyamid |
| Katalysator A (erf.) | 380 | blank | hoch |
| Katalysator B (V) | 270 | stark trüb | gering |

Vergleichsbeispiel = vergl., Erfindungsgemäß = erf.

Die Mischungen nach dem Stand der Technik weisen i.d.R. Topfzeiten von < 300s auf, was zu Inhomogenitäten und einer geringen Kristallinität des Gusspolyamids führt. Darüber hinaus polymerisieren die den Katalysator enthaltenden Schmelzen bereits in der Vorlage an und verstopfen so die der Gussform vorgeschalteten Filter.

Die Beispiele zeigen, dass der erfindungsgemäße Katalysator Topfzeiten von deutlich über 300s aufweist. Die erfindungsgemäßen Zusammensetzungen trüben zudem nicht ein.

Dadurch können auch großvolumige qualitativ hochwertige Gusspolyamid-Teile hergestellt werden. Weiterhin kann die Qualität der so hergestellten Gusspolyamide bezüglich Kristallinität bzw. Homogenität deutlich verbessert werden.

## Patentansprüche

1. Zusammensetzung enthaltend
a) mindestens ein Lactamat und
b) mindestens ein Salz einer mit Heteroatomen substituierten organischen Säure und/oder Ester einer mit Heteroatomen substituierten organischen Säure, und gegebenenfalls
c) ein Lactam oder eine Mischung aus mehreren Lactamen.

2. Zusammensetzung nach Anspruch 1, **dadurch gekennzeichnet, dass** es sich bei dem Lactamat um mindestens eine Verbindung ausgewählt aus der Gruppe der Alkali-Alumo-Dilactamate, Alkali- und/oder Erdalkalilactamate handelt.

3. Zusammensetzung nach einem oder mehreren der Ansprüche 1 bis 2, **dadurch gekennzeichnet, dass** es sich bei dem Ester b) um Alkylester, bevorzugt Methyl- und/oder Ethylester handelt.

4. Zusammensetzung nach einem oder mehreren der Ansprüche 1 bis 2, **dadurch gekennzeichnet, dass** das Salz der mit Heteroatomen, vorzugsweise mit Aminogruppen, substituierten organischen Säure b) 1 bis 12-Kohlenstoffatome, bevorzugt 4 bis 6 Kohlenstoffatomen hat.

5. Zusammensetzung nach einem oder mehreren der Ansprüche 1 bis 4, **dadurch gekennzeichnet, dass** es sich bei den Heteroatomen um Stickstoff, Schwefel, Phosphor, vorzugsweise Stickstoff und/oder Halogenide, vorzugsweise Chlor und/oder Brom, handelt.

6. Zusammensetzung nach Anspruch 5, **dadurch gekennzeichnet, dass** es sich bei dem Salz der mit Heteroatomen substituierten einer organischen Säure und/oder Ester einer mit Heteroatomen substituierten organischen Säure b) um Aminocapronate und/oder Aminolaurate, bevorzugt Alkali- und/ oder Erdalkaliaminocapronate und/oder -Laurate, besonders bevorzugt Natrium-, Kalium- und/oder Magnesium-Aminocapronat, handelt.

7. Zusammensetzung nach einem oder mehreren der Ansprüche 1 bis 6, **dadurch gekennzeichnet, dass** das Lactam der allgemeinen Formel 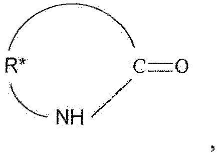 entspricht, wobei R* eine Alkylengruppe mit 3 bis 13 Kohlenstoffatomen darstellt.

8. Zusammensetzung nach einem oder mehreren der Ansprüche 1 bis 7, **dadurch gekennzeichnet, dass** die Bestandteile a) und b) im Verhältnis 3:1 bis 50: 1, bevorzugt 5:1 bis 40: 1, besonders bevorzugt 9:1 vorliegen und der Anteil an Lactam c), bezogen auf die Bestandteile a) und b) vorzugsweise 0 - 99 Gew.%, besonders bevorzugt 50 - 85 Gew.% beträgt.

9. Verfahren zur Herstellung von Zusammensetzung nach einem oder mehreren der Ansprüche 1 bis 8, **dadurch gekennzeichnet, dass** das Lactamat a) durch Umsetzung von mindestens einem Lactam, welches Lactam c) entsprechen, aber auch von diesem verschieden sein kann, mit Alkali- oder Erdalkali-Alkoholaten in Gegenwart von mindestens einem Salz einer Heteroatomen substituierten organischen Säure und/oder Ester einer mit Heteroatomen substituierten organischen Säure b) bei anschließender oder gleichzeitiger Entfernung des entstehenden Alkohols durch Destillation hergestellt wird und anschließend gegebenenfalls weiteres Lactam c) zugesetzt wird.

10. Verfahren zur Herstellung von Zusammensetzung nach Anspruch 9, **dadurch gekennzeichnet, dass** die Herstellung der Salze einer mit Heteroatomen substituierten organischen Säure und/oder Ester der mit Heteroatomen substituierten organischer Säuren in situ durch Zugabe von Säuren, Wasser und/oder Alkali- und/oder Erdalkalihydroxiden zu a) und gegebenenfalls c) erfolgt.

11. Gusspolyamide, erhältlich durch die Polymerisation von Lactamen mit mindestens einer Zusammensetzung der Ansprüche 1 bis 8 bei Temperaturen von 80 - 180°C gegebenenfalls in Anwesenheit von Aktivatoren und/oder weiteren Zusatz- und Hilfsstoffen.

12. Verfahren zur Herstellung von Gusspolyamiden, erhältlich durch die Polymerisation von Lactamen mit mindestens einer Zusammensetzung der Ansprüche 1 bis 8 bei Temperaturen von 80-120°C, vorzugsweise 120-160°C, in Anwesenheit von Aktivatoren und gegebenenfalls weiteren Zusatz- und Hilfsstoffen.

13. Verwendung der Zusammensetzung nach einem oder mehreren der Ansprüche 1 bis 8 als Katalysator für die Herstellung von Gusspolyamid.

14. Verwendung der Zusammensetzung nach einem oder mehreren der Ansprüche 1 bis 8 zur Herstellung von Rollen, vorzugsweise für Personenaufzüge und Halbzeuge, für Behälter, Zahnräder, Rohre, Stäbe und Platten für den Maschinenbau und die Automobilindustrie.

## Claims

1. Composition comprising
a) at least one lactamate and
b) at least one salt of an organic acid substituted by heteroatoms and/or ester of an organic acid substituted by heteroatoms, and optionally
c) a lactam or a mixture of two or more lactams.

2. Composition according to Claim 1, **characterized in that** the lactamate is at least one compound selected from the group of the alkali metal aluminodilactamates and alkali metal and/or alkaline earth metal lactamates.

3. Composition according to one or more of Claims 1 and 2, **characterized in that** the ester b) comprises alkyl esters, preferably methyl and/or ethyl esters.

4. Composition according to one or more of Claims 1 and 2, **characterized in that** the salt of the organic acid b) substituted by heteroatoms, preferably by amino groups, has 1 to 12 carbon atoms, preferably 4 to 6 carbon atoms.

5. Composition according to one or more of Claims 1 to 4, **characterized in that** the heteroatoms are nitrogen, sulfur, phosphorus, preferably nitrogen, and/or halides, preferably chlorine and/or bromine.

6. Composition according to Claim 5, **characterized in that** the salt of an organic acid substituted by heteroatoms and/or ester of an organic acid b) substituted by heteroatoms comprises aminocapronates and/or aminolaurates, preferably alkali metal and/or alkaline earth metal aminocapronates and/or -laurates, more preferably sodium aminocapronate, potassium aminocapronate and/or magnesium aminocapronate.

7. Composition according to one or more of Claims 1 to 6, **characterized in that** the lactam corresponds to the general formula 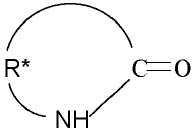 where R* is an alkylene group having 3 to 13 carbon atoms.

8. Composition according to one or more of Claims 1 to 7, **characterized in that** constituents a) and b) are present in a ratio of 3:1 to 50:1, preferably 5:1 to 40:1, more preferably 9:1, and the proportion of lactam c), based on constituents a) and b), is preferably 0-99% by weight, more preferably 50-85% by weight.

9. Process for preparing composition according to one or more of Claims 1 to 8, **characterized in that** the lactamate a) is prepared by reaction of at least one lactam which corresponds to or else may be different than lactam c) with alkali metal or alkaline earth metal alkoxides in the presence of at least one salt of an organic acid substituted by heteroatoms and/or ester of an organic acid b) substituted by heteroatoms with subsequent or simultaneous removal of the alcohol formed by distillation, and then further lactam c) is optionally added.

10. Process for preparing composition according to Claim 9, **characterized in that** the salts of an organic acid substituted by heteroatoms and/or esters of the organic acids substituted by heteroatoms are prepared in situ by addition of acids, water and/or alkali metal and/or alkaline earth metal hydroxides to a) and optionally c).

11. Cast polyamides obtainable by the polymerization of lactams with at least one composition of Claims 1 to 8 at temperatures of 80-180°C, optionally in the presence of activators and/or further additives and assistants.

12. Process for producing cast polyamides obtainable by the polymerization of lactams with at least one composition of Claims 1 to 8 at temperatures of 80-120°C, preferably 120-160°C, in the presence of activators and optionally further additives and assistants.

13. Use of the composition according to one or more of Claims 1 to 8 as a catalyst for the production of cast polyamide.

14. Use of the composition according to one or more of Claims 1 to 8 for production of rollers, preferably for passenger elevators and semifinished products, for vessels, gears, tubes, bars and sheets for mechanical engineering and the automobile industry.

## Revendications

1. Composition contenant :
a) au moins un lactamate et
b) au moins un sel d'un acide organique substitué avec des hétéroatomes et/ou un ester d'un acide organique substitué avec des hétéroatomes, et éventuellement
c) un lactame ou un mélange de plusieurs lactames.

2. Composition selon la revendication 1, **caractérisée en ce que** le lactamate consiste en au moins un composé choisi dans le groupe constitué par les alumodilactamates alcalins, les lactamates alcalins et/ou alcalino-terreux.

3. Composition selon une ou plusieurs des revendications 1 à 2, **caractérisée en ce que** l'ester b) consiste en un ester alkylique, de préférence un ester méthylique et/ou éthylique.

4. Composition selon une ou plusieurs des revendications 1 à 2, **caractérisée en ce que** le sel de l'acide organique substitué avec des hétéroatomes, de préférence avec des groupes amino, b) comprend 1 à 12 atomes de carbone, de préférence 4 à 6 atomes de carbone.

5. Composition selon une ou plusieurs des revendications 1 à 4, **caractérisée en ce que** les hétéroatomes consistent en l'azote, le soufre, le phosphore, de préférence l'azote et/ou des halogénures, de préférence le chlore et/ou le brome.

6. Composition selon la revendication 5, **caractérisée en ce que** le sel du d'un acide organique substitué avec des hétéroatomes et/ou l'ester d'un acide organique substitué avec des hétéroatomes b) consiste en des aminocapronates et/ou des aminolaurates, de préférence des aminocapronates et/ou -laurates alcalins et/ou alcalino-terreux, de manière particulièrement préférée l'aminocapronate de sodium, de potassium et/ou de magnésium.

7. Composition selon une ou plusieurs des revendications 1 à 6, **caractérisée en ce que** le lactame correspond à la formule générale 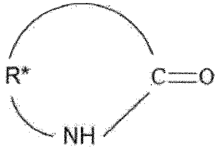 dans laquelle R* représente un groupe alkylène de 3 à 13 atomes de carbone.

8. Composition selon une ou plusieurs des revendications 1 à 7, **caractérisée en ce que** les constituants a) et b) sont présents en un rapport de 3:1 à 50:1, de préférence de 5:1 à 40:1, de manière particulièrement préférée de 9:1, et la proportion de lactame c), par rapport aux constituants a) et b), est de préférence de 0 à 99 % en poids, de manière particulièrement préférée de 50 à 85 % en poids.

9. Procédé de fabrication d'une composition selon une ou plusieurs des revendications 1 à 8, **caractérisé en ce que** le lactamate a) est fabriqué par mise en réaction d'au moins un lactame, qui peut correspondre au lactame c), mais également être différent de celui-ci, avec des alcoolates alcalins ou alcalino-terreux en présence d'au moins un sel d'un acide organique substitué avec des hétéroatomes et/ou un ester d'un acide organique substitué avec des hétéroatomes b) avec élimination ultérieure ou simultanée de l'alcool formé par distillation, puis un lactame c) supplémentaire est éventuellement ajouté.

10. Procédé de fabrication d'une composition selon la revendication 9, **caractérisé en ce que** la fabrication des sels d'un acide organique substitué avec des hétéroatomes et/ou des esters d'un acide organique substitué avec des hétéroatomes a lieu in situ par ajout d'acides, d'eau et/ou d'hydroxydes alcalins et/ou alcalino-terreux à a) et éventuellement c).

11. Polyamides coulés, pouvant être obtenus par polymérisation de lactames avec au moins une composition selon les revendications 1 à 8 à des températures de 80 à 180 °C éventuellement en présence d'activateurs et/ou d'autres additifs et adjuvants.

12. Procédé de fabrication de polyamides coulés, pouvant être obtenus par polymérisation de lactames avec au moins une composition selon les revendications 1 à 8 à des températures de 80 à 120 °C, de préférence de 120 à 160 °C, en présence d'activateurs et éventuellement d'autres additifs et adjuvants.

13. Utilisation de la composition selon une ou plusieurs des revendications 1 à 8 en tant que catalyseur pour la fabrication d'un polyamide coulé.

14. Utilisation de la composition selon une ou plusieurs des revendications 1 à 8 pour la fabrication de rouleaux, de préférence pour des ascenseurs, et de produits semi-finis, pour des contenants, des roues dentées, des tubes, des tiges et des plaques pour la construction de machines et l'industrie automobile.
